Europäisches Patentamt

European Patent Office

Office européen des brevets

(19)

(11) Veröffentlichungsnummer : **0 222 988**
**B1**

(12)

# EUROPÄISCHE PATENTSCHRIFT

(45) Veröffentlichungstag der Patentschrift :
**17.08.88**

(21) Anmeldenummer : **86111694.5**

(22) Anmeldetag : **23.08.86**

(51) Int. Cl.⁴ : **C 07 C 31/133, C 07 C 29/136**

(54) **Verfahren zur Herstellung von Hydroxymethylcyclopropan (Cyclopropylmethanol).**

(30) Priorität : **26.10.85 DE 3538132**

(43) Veröffentlichungstag der Anmeldung :
**27.05.87 Patentblatt 87/22**

(45) Bekanntmachung des Hinweises auf die Patenterteilung : **17.08.88 Patentblatt 88/33**

(84) Benannte Vertragsstaaten :
**AT BE CH DE FR GB IT LI NL SE**

(56) Entgegenhaltungen :
**US-A- 2 340 688**

(73) Patentinhaber : **HÜLS AKTIENGESELLSCHAFT**
**- RSP Patente / PB 15 - Postfach 13 20**
**D-4370 Marl 1 (DE)**

(72) Erfinder : **Otte, Werner, Dr.**
**Eichenstück 55**
**D-4270 Dorsten 11 (DE)**
Erfinder : **Nehring, Rudolf, Dr.**
**Griesheimer Strasse 12**
**D-4370 Marl (DE)**

**Beschreibung**

Die Erfindung betrifft ein Verfahren zur selektiven Herstellung von Hydroxymethylcyclopropan aus Estern der Cyclopropancarbonsäure in Gegenwart handelsüblicher Zinkchromit-Katalysatoren. Hydroxymethylcyclopropan ist eine bekannte Chemikalie, die als Zwischenprodukt zur Synthese, beispielsweise von Baktericiden, Fungiciden, Herbiciden und Insekticiden verwendet wird.

Nach bekannten Verfahren erhält man Hydroxymethylcyclopropan aus Cyclopropancarbonsäure in Gegenwart von Lithiumalanat (Beilstein E IV 6, S. 4) oder durch Umsetzungen von Cyclopropancarbonsäuren mit metallorganischen Verbindungen in Gegenwart elektrophiler Verbindungen (US-PS 4 085 173, US-PS 4 085 273, US-PS 4 054 480, US-PS 3 998 899, US-PS 3 959 324) oder durch anodische Oxidation von Cyclobutancarbonsäure (J. Am. Chem. Soc. 82, S. 2645 bis 2646 (1960)).

Diese Verfahren sind recht aufwendig und bedürfen zu ihrer Ausführung einiger komplizierter und teurer Chemikalien, die in ihrer Handhabung nicht unkritisch sind, außerdem sind die Ausbeuten nur mäßig.

Es bestand daher die Aufgabe, ein wirtschaftliches Verfahren zur Herstellung von Hydroxymethylcyclopropan zu finden, das auf den Einsatz teurer und nicht leicht zu handhabender Chemikalien verzichtet sowie das Hydroxymethylcyclopropan in hoher Ausbeute ergibt.

Diese Aufgabe wird erfindungsgemäß entsprechend den Angaben der Patentansprüche gelöst.

Überraschenderweise wurde gefunden, daß bei dem erfindungsgemäßen Verfahren Hydroxymethylcyclopropan in nahezu quantitativer Ausbeute bei annähernd vollständigem Umsatz erhalten wird.

Das erfindungsgemäße Verfahren wird bei einer Temperatur von 200 bis 350 °C, vorzugsweise im Temperaturbereich von 250 bis 300 °C, insbesondere von 240 bis 300 °C durchgeführt.

Ferner wendet man bei dem erfindungsgemäßen Verfahren einen Wasserstoffdruck oberhalb von 200 bar, von 200 bis 320 bar, vorzugsweise von 240 bis 300 bar, an. Als Einsatzprodukt eignen sich insbesondere die Cyclopropancarbonsäureester, deren Alkoholkomponente 1 bis 10, vorzugsweise 1 bis 8 C-Atome, aufweisen.

Im allgemeinen wird das erfindungsgemäße Verfahren ohne Verwendung eines Lösemittels durchgeführt, jedoch ist die Anwesenheit eines Lösemittels nicht schädlich.

Bei dem erfindungsgemäßen Verfahren wird ein handelsüblicher Zinkchromit-Katalysator (z. B. BASF S5-10) eingesetzt. Geeignete Zinkchromit-Katalysatoren enthalten im allgemeinen 40 bis 80 Gew.-% ZnO und 10 bis 40 Gew.-% $Cr_2O_3$. Die üblicherweise zur Hydrierung von Carbonsäureestern eingesetzten CuCr-Kontakte führen bei nahezu vollständigem Umsatz überwiegend zu n-Butanol und nur zu wenig Hydroxymethylcyclopropan.

Das erfindungsgemäße Verfahren kann sowohl diskontinuierlich als auch kontinuierlich in üblicher Weise ausgeführt werden, z. B. in einem Rührautoklaven oder einem Reaktionsrohr. Die zur Hydrierung erforderlichen Apparaturen sind Stand der Technik. Das erfindungsgemäße Verfahren führt man in der Sumpfphase oder Rieselphase durch.

Bei diskontinuierlich betriebenem Verfahren wird üblicherweise in der Sumpfphase in einem Autoklaven in Gegenwart eines pulverförmigen Katalysators gearbeitet. Besonders vorteilhaft läßt sich das erfindungsgemäße Verfahren kontinuierlich ausführen. Dies erfolgt in üblicher Weise dadurch, daß man das flüssige Ausgangsprodukt über den im Reaktionsrohr befindlichen stückig angeordneten Katalysator, nach dem Rieselphasenprinzip, führt, während der Wasserstoff im Gleich- oder Gegenstrom durch das Reaktionsrohr geleitet wird. Vorteilhafterweise wird der überschüssige Wasserstoff im Kreis geführt.

Die besonderen Vorteile des erfindungsgemäßen Verfahrens liegen in der Einfachheit der Durchführung und der Wirtschaftlichkeit.

Beispiel 1

Durch ein Reaktionsrohr, gefüllt mit 400 ml Zn-Chromit-Kontakt, werden stündlich 100 ml Cyclopropancarbonsäuremethylester und 400 Nl Wasserstoff geführt. Die Temperatur beträgt 270 °C und der Druck 300 bar. Im Hydrieraustrag sind enthalten.

| | |
|---|---|
| Vorläufe | 0,2 % |
| n-Butanol | 0,5 % |
| Hydroxymethylcyclopropan | 66,6 % |
| Methanol | 30,5 % |
| Cyclopropancarb.-ester | 0,2 % |
| Sa. Zwischenläufe | 1,5 % |
| Rest | 0,5 % |

Beispiel 2

Ausführung wie Beispiel 1, jedoch wird Cyclopropancarbonsäure-n-butyl-ester eingesetzt. Der

2

**0 222 988**

Hydrieraustrag setzt sich wie folgt zusammen :

| | |
|---|---|
| Vorläufe | 0,3 % |
| n-Butanol | 50,3 % |
| Hydroxymethylcyclopropan | 47,4 % |
| Ester | 0,1 % |
| Sa. Zwischenläufe | 1,3 % |
| Rest | 0,6 % |

## Beispiel 3

Ausführung wie Beispiel 1, jedoch wird Cyclopropancarbonsäure-2-ethylhexylester eingesetzt. Der Hydrieraustrag hat folgende Zusammensetzung :

| | |
|---|---|
| Vorläufe | 0,2 % |
| n-Butanol | 0,4 % |
| Hydroxymethylcyclopropan | 34,1 % |
| 2-Ethylhexanol | 64,0 % |
| Ester | 0,2 % |
| Sa. Zwischenläufe | 0,9 % |
| Rest | 0,2 % |

## Beispiel 4

Ausführung wie Beispiel 3, jedoch wird die Temperatur auf 300 °C erhöht. Der Hydrieraustrag hat folgende Zusammensetzung :

| | |
|---|---|
| Vorläufe | 0,4 % |
| n-Butanol | 1,9 % |
| Hydroxymethylcyclopropan | 30,5 % |
| 2-Ethylhexanol | 64,3 % |
| Ester | größer 0,1 % |
| Sa. Zwischenläufe | 2,4 % |
| Rest | 0,5 % |

## Beispiel 5

Ausführung wie Beispiel 3, jedoch wird die Temperatur auf 250 °C erniedrigt. Der Hydrieraustrag hat folgende Zusammensetzung :

| | |
|---|---|
| Vorläufe | 0,1 % |
| n-Butanol | 0,3 % |
| Hydroxymethylcyclopropan | 32,4 % |
| 2-Ethylhexanol | 62,3 % |
| Ester | 4,1 % |
| Sa. Zwischenläufe | 0,6 % |
| Rest | 0,2 % |

## Vergleichsbeispiel

Ausführung wie Beispiel 1, jedoch wird anstelle eines ZnCr- ein CuCr-Katalysator (HARSHAW 1107) eingesetzt und die Reaktionstemperatur auf 210 °C gesenkt. Der Hydrieraustrag hat folgende Zusammensetzung :

| | |
|---|---|
| Vorläufe | 2,8 % |
| Methanol | 30,7 % |
| n-Butanol | 59,3 % |
| Hydroxymethylcyclopropan | 4,9 % |
| Ester | 0,2 % |
| Zwischenläufe | 1,2 % |
| Rest | 0,9 % |

Die in den Beispielen angegebenen Konzentrationen sind durch Gaschromatographie bestimmt und beziehen sich auf Flächenprozent.

3

**0 222 988**

**Patentansprüche**

1. Verfahren zur selektiven Herstellung von Hydroxymethylcyclopropan aus Cyclopropancarbonsäureestern, dadurch gekennzeichnet, daß man die Cyclopropancarbonsäureester in Gegenwart eines Zn-Chromit-Katalysators in der Sumpf- und Rieselphase bei einem Wasserstoffdruck von 200 bis 320 bar und Temperaturen von 200 bis 350 °C hydriert.

2. Verfahren nach Anspruch 1, dadurch gekennzeichnet, daß man bei einem Wasserstoffdruck von 240 bis 300 bar hydriert.

3. Verfahren nach Anspruch 1 und 2, dadurch gekennzeichnet, daß man bei Temperaturen von 200 bis 300 °C, vorzugsweise 240 bis 300 °C, hydriert.

4. Verfahren nach Anspruch 1 bis 3, dadurch gekennzeichnet, daß man Cyclopropancarbonsäureester einsetzt, deren Alkoholkomponente 1 bis 10, vorzugsweise 1 bis 8, Kohlenstoffatome besitzt.

5. Verfahren nach Anspruch 1 bis 4, dadurch gekennzeichnet, daß man Cyclopropancarbonsäuremethylester hydriert.

6. Verfahren nach Anspruch 1 bis 4, dadurch gekennzeichnet, daß man Cyclopropancarbonsäure-n-butylester hydriert.

7. Verfahren nach Anspruch 1 bis 4, dadurch gekennzeichnet, daß man Cyclopropancarbonsäure-2-ethylhexylester hydriert.

**Claims**

1. A process for the selective preparation of hydroxymethylcyclopropane from a cyclopropanecarboxylic acid ester, characterised in that the cyclopropanecarboxylic acid ester is hydrogenated in the presence of a zinc chromite catalyst in the bottom phase and trickle phase under a hydrogen pressure of 200 to 320 bar and temperature of 200 to 350 °C.

2. A process according to claim 1, characterised in that hydrogenation is performed under a hydrogen pressure of 240 to 300 bar.

3. A process according to claim 1 or 2, characterised in that hydrogenation is performed at a temperature of 200 to 300 °C, preferably 240 to 300 °C.

4. A process according to any of claims 1 to 3, characterised in that a cyclopropanecarboxylic acid ester of which the alcohol component possesses 1 to 10, preferably 1 to 8, carbon atoms, is employed.

5. A process according to any of claims 1 to 4, characterised in that methyl cyclopropanecarboxylate is hydrogenated.

6. A process according to any of claims 1 to 4, characterised in that n-butyl cyclopropanecarboxylate is hydrogenated.

7. A process according to any of claims 1 to 4, characterised in that 2-ethylhexyl cyclopropanecarboxylate is hydrogenated.

**Revendications**

1. Procédé de préparation sélective d'hydroxyméthylcyclopropane à partir d'esters de l'acide cyclopropane-carboxylique, caractérisé par le fait que l'on hydrogène les esters de l'acide cyclopropane-carboxylique en présence d'un catalyseur au chromite de zinc en phase pâteuse et en phase de ruissellement sous une pression d'hydrogène de 200 à 320 bars et à des températures de 200 à 350 °C.

2. Procédé selon la revendication 1, caractérisé par le fait que l'on procède à l'hydrogénation sous une pression d'hydrogène de 240 à 300 bars.

3. Procédé selon les revendications 1 et 2, caractérisé par le fait que l'on procède à l'hydrogénation à des températures de 200 à 300 °C, de préférence de 240 à 300 °C.

4. Procédé selon les revendications 1 à 3, caractérisé par le fait que l'on utilise des esters de l'acide de cyclopropane-carboxylique dont le composant alcool possède de 1 à 10, de préférence de 1 à 8, atomes de carbone.

5. Procédé selon les revendications 1 à 4, caractérisé par le fait que l'on hydrogène du cyclopropane-carboxylate de méthyle.

6. Procédé selon les revendications 1 à 4, caractérisé par le fait que l'on hydrogène du cyclopropanecarboxylate de n-butyle.

7. Procédé selon les revendications 1 à 4, caractérisé par le fait que l'on hydrogène du cyclopropane-carboxylate de 2-éthylhexyle.